# EUROPEAN PATENT APPLICATION

(11) **EP 4 338 585 A1**
(43) Date of publication of application: **20.03.2024**
(21) Application number: 22807523.0
(22) Date of filing: 12.05.2022
(51) Int. Cl.: A01H 6/02, A01H 1/00, C12Q 1/68, C12Q 1/6895

(54) **SPINACH PLANT HAVING NOVEL DOWNY MILDEW-RESISTANT GENE**

(30) Priority: 12.05.2021 JP 2021080911
(71) Applicant: Sakata Seed Corporation, Yokohama-shi Kanagawa 224-0041 (JP)
(72) Inventor: SUGIHARA Yuichi, Yokohama-shi, Kanagawa 224-0041 (JP); NAKAMURA Yo, Yokohama-shi, Kanagawa 224-0041 (JP); KIMURA Ryo, Yokohama-shi, Kanagawa 224-0041 (JP); MORITAMA Yosuke, Yokohama-shi, Kanagawa 224-0041 (JP)
(74) Representative: Bandpay & Greuter
(86) International application number: PCT/JP2022/020033
(87) International publication number: WO 2022/239824

(57) **Abstract**

A downy mildew resistant spinach plant having a downy mildew resistance RTM-1 gene which is located on chromosome 4 of a Spinacia tetrandra line CGN25466:MGK 01, the above downy mildew resistant spinach plant in which the RTM-1 gene is a gene that exists in a range from chr4_7962907 to chr4_8617232 on chromosome 4 of the Spinacia tetrandra line CGN25466:MGK 01, and either of the above downy mildew resistant spinach plants wherein the plant is resistant to at least the downy mildew races Pfs1, Pfs2, Pfs3, Pfs4, Pfs5, Pfs6, Pfs7, Pfs8, Pfs9, Pfs10, Pfs11, Pfs12, Pfs13, Pfs14, Pfs15, Pfs16, Pfs17, Pfs18 and Pfs19, and the race indicated by UA1014 strain.

## Description

### [Technical Field]

The present invention relates to a spinach plant having a gene that exhibits resistance to a broad range of races of downy mildew, and also relates to a method for producing the spinach plant.

Priority is claimed on Japanese Patent Application No. 2021-080911, filed May 12, 2021, the content of which is incorporated herein by reference.

### [Background Art]

Spinach (Spinacia oleracea L.) is an annual or perennial plant belonging to the genus Spinacia of the family Amaranthaceae that is native to western Asia and widely cultivated. It is thought to have first been brought to Japan from China in the Edo Period. Generally, the radical leaves (rosette) of spinach are used for food consumption, and because the levels of vitamins, iron and calcium in spinach are particularly high, the nutritional value is very high. In recent years, for reasons of nutrient level and convenience, the worldwide market for baby spinach leaves has grown rapidly. Consequently, spinach is now one of the world's most important vegetables.

Plant species are generally either phenotypically uniform open pollinated lines or first filial generation hybrid (hereinafter abbreviated as "F1") varieties, and for most major crop species, F1 varieties are more prevalent. F1 varieties tend to exhibit more vigorous growth due to heterosis. As a result, F1 varieties offer considerable advantages, including rapid growth and increased yield, and can also be expected to exhibit improved environmental adaptability such as resistance to pests and better tolerance to cold and extreme heat. Further, although being heterozygous, because individuals of an F1 variety possess the same genotype, their phenotype exhibits an extremely high degree of uniformity. Accordingly, the marketability of the harvested product is enhanced. Moreover, because useful traits governed by dominant genes can be accumulated in the parent lines, faster breeding of desirable varieties is possible. Because of the type of superior characteristics described above, F1 varieties now represent the majority of cultivated varieties among the major crops. Similarly, in the case of spinach grown for consumption, although phenotypically uniform open pollinated lines were predominant until the 1960s, nowadays almost all cultivated spinach is F1 varieties.

One of the diseases that damages spinach is downy mildew, which is caused by the fungal pathogen Peronospora farinosa f. sp. spinaciae (commonly abbreviated as Pfs). Downy mildew is one of the most serious spinach diseases, it spreads rapidly and has an extremely severe effect on the harvested product quality. Countermeasures that have been attempted for downy mildew include control of the pathogenic organism, either by cultural control or chemical control using agricultural chemicals or the like, but the use of resistant varieties has proven the most effective method in terms of environmental impact, cultivation labor, and cost and the like.

Downy mildew is known to exhibit rapid development of new pathogenic races , withsuch new races being able to evade or overcome the heritable resistance traits of varieties that were previously resistant to all known races. The International Working Group on Peronospora in spinach (IWGP) is a consortium of seed companies that receives research support from the University of Arkansas and the University of California (USA) and the Netherlands Inspection Service for Horticulture (Naktuinbouw). The IWGP members monitor the appearance and spread of new races of downy mildew and assigns formal names to those races. Since the first report of a downy mildew outbreak in 1824, 19 races of downy mildew have been named to date (Non-Patent Document 1).

To adapt to this continual appearance of new races, the search for novel resistant materials is extremely important in the breeding of spinach. The search for downy mildew resistant genetic material is occurring not only among cultivated varieties, but also within related wild species. For example, according to the Center for Genetic Resources, the Netherlands (CGN), searches for downy mildew resistance genetic material were conducted in 2008 within the species Spinacia turkestanica, and in 2011 within the species Spinacia tetrandra. Further, in 2011, Correll et al. reported that six types of genes known as RPF controlled known downy mildew resistance (Non-Patent Document 2). Moreover, RPF1 to RPF10, RPF11 (Patent Document 1), RPF12 (Patent Document 2), RPF13 (Patent Document 3), RPF14 (Patent Document 4), RPF15 (Patent Document 5), R6 (Patent Document 6), and R15 (Patent Document 7) and the like have all been reported as downy mildew resistance genes. By introducing a downy mildew resistance gene derived from a wild species related to spinach into the cultivated spinach species Spinacia oleracea L., a spinach having superior downy mildew resistance can be developed (Patent Document 8).

RPF genes are a plurality of alleles, or a plurality of closely linked genes, that exist at one gene locus known as the RPF gene locus. In an F1 variety, by incorporating two different RFP alleles, plant breeders have been able to be achieved broad downy mildew race resistance (Non-Patent Document 3, Patent Document 5). For example, the RPF1 gene, the RPF2 gene and the RPF3 gene are located on chromosome 3 (Non-Patent Document 3), and the RPF15 gene is also located on chromosome 3 (Patent Document 5). Further, a downy mildew resistance gene disclosed in Patent Document 8 is stated to be located on linkage group 6, but according to sequence information, it is actually located on chromosome 3 (Patent Document 5).

A single WOLF gene or two adjacent WOLF genes, each of which is broadly classified as either an alpha WOLF gene or a beta WOLF gene depending on the structure, exist at the downy mildew resistance locus (Patent Document 9). The alpha WOLF gene and the beta WOLF gene each include a plurality of alleles that impart a specific resistance profile, and the LRR domain sequence of each WOLF gene and the resistance pattern of each gene against various downy mildew races have been disclosed. In theory, combining WOLF genes having different resistance patterns should enable the design of a desired resistance pattern. However, with conventional cross breeding, arbitrarily combining extremely closely linked genes is practically impossible. Accordingly, until now, RPF genes have been treated jointly as a single gene.

Moreover, variants that impart the desired resistance pattern can also be developed by introducing a downy mildew resistance gene by genetic transformation (genetic recombination), or by endogenous gene editing. However, crops created by genetic recombination or gene editing suffer from the problem of still not being widely accepted by the general public.

The downy mildew resistance gene p10 is located on chromosome 1, and has been reported to exhibit resistance to Pfs1, Pfs2, Pfs3, Pfs4, Pfs5, Pfs6, Pfs7, Pfs8, Pfs9, Pfs10, Pfs11, Pfs12, Pfs13, Pfs14, Pfs15 and Pfs16. However, the resistance imparted by the p10 gene is only expressed when homozygous, and the degree of resistance is only moderate (Patent Document 10). As a result, it is surmised that breeding resistant varieties using the p10 gene is not only more difficult than using genes having dominant resistance expression, but also results in a degree of resistance that is insufficient from a practical perspective. Furthermore, the Spinacia tetrandra line CGN25474:MGK 18:RNR120251 (hereinafter abbreviated as CGN25474:MGK 18) has a downy mildew resistance gene located on chromosome 4, and has been reported to exhibit resistance to Pfs4, Pfs7, Pfs9, Pfs10, Pfs11, Pfs12, Pfs13, Pfs14, Pfs15, Pfs16 and Pfs17 (Patent Document 11).

### [Citation List]

### [Patent Documents]

[Patent Document 1]
   U.S. Patent Publication No. 10,258,001
[Patent Document 2]
   U.S. Patent Publication No. 10,258,002
[Patent Document 3]
   International Patent Publication No. WO2015/036378
[Patent Document 4]
   International Patent Publication No. WO2019/145446
[Patent Document 5]
   International Patent Publication No. WO2019/145447
[Patent Document 6]
   Japanese Patent (Granted) Publication No. 6457269
[Patent Document 7]
   U.S. Patent Publication No. 9,974,276
[Patent Document 8]
   Japanese Patent (Granted) Publication No. 6684207
[Patent Document 9]
   International Patent Publication No. WO2018/059651
[Patent Document 10]
   U.S. Patent Application No. 2019/0104700
[Patent Document 11]
   International Patent Publication No. WO2020/239215

### [Non Patent Documents]

[Non Patent Document 1]
   Ribera et al., Euphytica, 2020, 216:48.
[Non Patent Document 2]
   Correll et al., European Journal of Plant Pathology, 2011, vol. 129, pp. 193-205.
[Non Patent Document 3]
   Feng et.al, Euphytica, 2018, 214:174.
[Non Patent Document 4]
   International Seed Federation, "Differential Sets Peronospora farinosa f. sp. spinaciae (P. effusa)", 2021, <online> https://worldseed.org/wp-content/uploads/2021/11/20210608_DRTWG_Peronospora-effusa.pdf
[Non Patent Document 5]
   Iwata and Ninomiya, Breeding Science, 2006, vol. 56(4), pp. 371-377.

### [Summary of Invention]

### [Technical Problem]

Existing downy mildew resistance lines mainly utilize an RPF gene on chromosome 3. On the other hand, spinach breeding usually requires that the parents of F1 varieties are genetically fixed to a high degree. As a result, a single parent may only have one resistance gene at the same site or extremely close sites. In other words, F1 varieties obtained by crossing different parents can only have two types of RPF genes located at the same site or extremely close sites on chromosome 3. Because there is no known RPF gene that exhibits resistance to all formally named races, to develop an F1 variety that exhibits resistance to a broad range of races, parent lines having a combination of RPF genes that offer mutually complementary resistance must be combined as the parents of a hybrid. This has become a significant limitation on the selection of the parents.

For example, if resistance genes that exist on chromosomes other than chromosome 3 could be utilized, then a larger effect could be expected by accumulating downy mildew resistance genes. In addition, if these resistance genes located on chromosomes other than chromosome 3 exhibit polymorphism, then further diversification of the downy mildew resistance genes can be promoted, enabling better suppression of the emergence of new races of downy mildew.

Accordingly, the present invention has been developed in light of the above types of problems associated with existing downy mildew resistance lines and F1 varieties, and it proposes a novel downy mildew resistance gene which does not exist on chromosome 3, exhibits resistance to a broader range of races than existing resistance genes, and is different from already reported resistance genes that exist on chromosome 4. In other words, the present invention provides a spinach plant having a novel downy mildew resistance gene that exhibits resistance to a broad range of races, a method for producing the spinach plant, and a method for producing spinach F1 plants or seeds thereof that utilize a plant line imparted with downy mildew resistance attributable to this gene.

### [Solution to Problem]

As a result of intensive research conducted in order to achieve the above objects, the inventors of the present invention discovered that a dominant downy mildew resistance gene that exhibits resistance to a broad range of races exists on chromosome 4 of the Spinacia tetrandra line CGN25466:MGK 01:RNR120234 (hereinafter abbreviated as CGN25466:MGK 01). Moreover, the inventors also discovered that by conducting interspecific crossing between a wild species of spinach having this downy mildew resistance gene and a cultivated variety of spinach, to obtain an F1 individual having downy mildew resistance, and then repeating crossing of this F1 individual with a cultivated variety of spinach (backcrossing), a spinach plant having the downy mildew resistance attributable to the wild species' downy mildew resistance gene, and having characteristics similar to the cultivated variety could be developed, enabling them to complete the present invention.

In other words, the present invention is as described below.
[1] A downy mildew resistant spinach plant (excluding Spinacia tetrandra) having a downy mildew resistance RTM-1 gene, which is located on chromosome 4 of the Spinacia tetrandra line CGN25466:MGK 01.
[2] The downy mildew resistant spinach plant according to [1] above, wherein the RTM-1 gene is a gene that exists in a range from chr4_7962907 to chr4_8617232 on chromosome 4 of the Spinacia tetrandra line CGN25466:MGK 01.
[3] The downy mildew resistant spinach plant according to [1] or [2] above wherein at least one allele of chromosome 4 contains a fragment that includes a range from chr4_8488603 to chr4_8510715 on chromosome 4 of the Spinacia tetrandra line CGN25466:MGK 01,
[4] The downy mildew resistant spinach plant according to any one of [1] to [3] above, wherein the RTM-1 gene is either homozygous or heterozygous.
[5] The downy mildew resistant spinach plant according to any one of [1] to [4] above, wherein the plant is resistant to at least downy mildew races Pfs1, Pfs2, Pfs3, Pfs4, Pfs5, Pfs6, Pfs7, Pfs8, Pfs9, Pfs10, Pfs11, Pfs12, Pfs13, Pfs14, Pfs15, Pfs16, Pfs17, Pfs18 and Pfs19, and a race indicated by UA1014 strain..
[6] The downy mildew resistant spinach plant according to any one of [1] to [5] above, wherein in at least one allele of chromosome 4:
   a SNP specified by chr4_8488603 is cytosine,
   a SNP specified by chr4_8494600 is thymine, or
   a SNP specified by chr4_8510715 is guanine.
[7] The downy mildew resistant spinach plant according to any one of [1] to [5] above, wherein in at least one allele of chromosome 4:
   a SNP specified by chr4_7962907 is guanine,
   a SNP specified by chr4_8152986 is cytosine,
   a SNP specified by chr4_8190990 is guanine,
   a SNP specified by chr4_8488603 is cytosine,
   a SNP specified by chr4_8494600 is thymine,
   a SNP specified by chr4_8510715 is guanine, or
   a SNP specified by chr4_8617232 is guanine.
[8] The downy mildew resistant spinach plant according to any one of [1] to [7] above, wherein the downy mildew resistant spinach plant is derived from an interspecific hybrid plant of Spinacia tetrandra and a cultivated variety of spinach.
[9] The downy mildew resistant spinach plant according to any one of [1] to [8] above, having downy mildew resistance derived from a plant specified in accession number FERM BP-22404 (TNKH-1 line).
[10] The downy mildew resistant spinach plant according to any one of [1] to [8] above, having downy mildew resistance derived from a plant specified in accession number FERM BP-22405 (TNKH-2 line).
[11] The downy mildew resistant spinach plant according to any one of [1] to [10] above, having at least one type of downy mildew resistance gene besides the RTM-1 gene.
[12] A downy mildew resistant spinach plant that is a downy mildew resistant spinach plant specified in accession number FERM BP-22404 (TNKH-1 line), a hybrid plant obtained using the downy mildew resistant spinach plant as a parent, or a progeny of either of these plants.
[13] A downy mildew resistant spinach plant that is a downy mildew resistant spinach plant specified in accession number FERM BP-22405 (TNKH-2 line), a hybrid plant obtained using the downy mildew resistant spinach plant as a parent, or a progeny of either of these plants.
[14] A method for predicting downy mildew resistance of a spinach plant, the method comprising investigating genotypes of SNPs from chr4_8488603 to chr4_8510715 of chromosome 4 of a test spinach plant, and predicting that the test spinach plant will have a high probability of exhibiting downy mildew resistance in those cases where, in at least one allele:
   a SNP specified by chr4_8488603 is cytosine,
   a SNP specified by chr4_8494600 is thymine, or
   a SNP specified by chr4_8510715 is guanine.
[15] A method for screening downy mildew resistant spinach plants, the method comprising investigating genotypes of SNPs from chr4_8488603 to chr4_8510715 of chromosome 4 of a test spinach plant, and selecting the test spinach plant as a downy mildew resistant spinach plant in those cases where, in at least one allele:
   a SNP specified by chr4_8488603 is cytosine,
   a SNP specified by chr4_8494600 is thymine, or
   a SNP specified by chr4_8510715 is guanine.
[16] A method for producing a downy mildew resistant spinach plant, the method having:
   a first crossing step of crossing a spinach plant having an RTM-1 gene and an arbitrary spinach plant,
   a second crossing step of obtaining a segregating population by subjecting an F1 individual obtained in the first crossing step to self-pollination, backcrossing, or interspecific crossing or intraspecific crossing with a spinach plant different from the parent used in the first crossing step, and
   a selection step of selecting a spinach plant having an RTM-1 gene from the segregating population.
[17] The method for producing a downy mildew resistant spinach plant according to [16] above, wherein the spinach plant having an RTM-1 gene is Spinacia tetrandra or a downy mildew resistant spinach plant according to any one of [1] to [13].
[18] The method for producing a downy mildew resistant spinach plant according to [16] or [17] above, wherein the arbitrary spinach plant, or the parent used in the interspecific crossing or intraspecific crossing in the second crossing step is a spinach plant having at least one type of downy mildew resistance gene besides the RTM-1 gene.
[19] A portion of a plant body of the downy mildew resistant spinach plant according to any one of [1] to [13].
[20] A leaf of the downy mildew resistant spinach plant according to any one of [1] to [13].
[21] A seed of the downy mildew resistant spinach plant according to any one of [1] to [13].
[22] A DNA marker for selecting a spinach plant having an RTM-1 gene, wherein the DNA marker is composed of at least one SNP selected from the group consisting of a SNP specified by chr4_8488603 of the spinach plant, a SNP specified by chr4_8494600 of the spinach plant, a SNP specified by chr4_8510715 of the spinach plant, and SNPs strongly linked to these SNPs.
[23] A kit which is used for selecting a spinach plant having an RTM-1 gene, the kit comprising at least one type of primer set selected from the group consisting of:
   a primer set for identifying a genotype of a SNP specified by chr4_8488603 of the spinach plant,
   a primer set for identifying a genotype of a SNP specified by chr4_8494600 of the spinach plant, and
   a primer set for identifying a genotype of a SNP specified by chr4_8510715 of the spinach plant.
[24] The kit according to [23] above, wherein
   the primer set for identifying a genotype of the SNP specified by chr4_8488603 includes a primer composed of a nucleotide sequence represented by SEQ ID NO: 13, a primer composed of a nucleotide sequence represented by SEQ ID NO: 14, and a primer composed of a nucleotide sequence represented by SEQ ID NO: 15,
   the primer set for identifying a genotype of the SNP specified by chr4_8494600 includes a primer composed of a nucleotide sequence represented by SEQ ID NO: 16, a primer composed of a nucleotide sequence represented by SEQ ID NO: 17, and a primer composed of a nucleotide sequence represented by SEQ ID NO: 18, and
   the primer set for identifying a genotype of the SNP specified by chr4_8510715 includes a primer composed of a nucleotide sequence represented by SEQ ID NO: 19, a primer composed of a nucleotide sequence represented by SEQ ID NO: 20, and a primer composed of a nucleotide sequence represented by SEQ ID NO: 21.

### [Advantageous Effects of Invention]

According to the present invention, a downy mildew resistant spinach plant that exhibits resistance to a broad range of races can be provided.

Further, by using the downy mildew resistant spinach plant according to the present invention as a parent, a novel spinach line that exhibits resistance to a broad range of races can be developed.

### [Brief Description of Drawings]

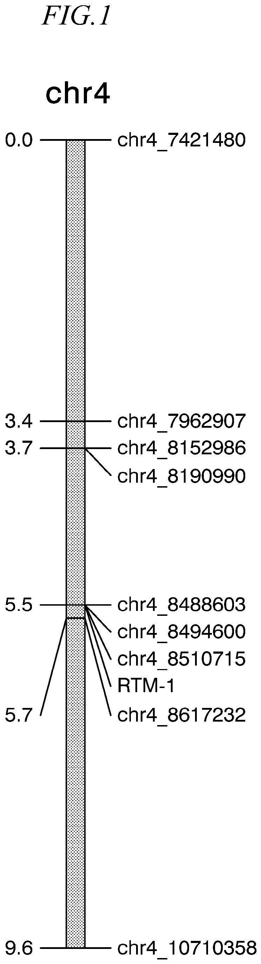

FIG. 1 is a linkage map in the vicinity of the RTM-1 gene locus of chromosome 4 of a spinach in Example 6.

### [Description of Embodiments]

In the present invention and the present description, a spinach plant describes a plant that is classified as belonging to the genus Spinacia. Examples of wild species of spinach include Spinacia tetrandra and Spinacia turkestanica and the like. Further, examples of cultivated species of spinach include Spinacia oleracea L.. In the present invention and the present description, these "cultivated species" describe plants of varieties used in cultivation, and include not only phenotypically uniform open pollinated lines, but also F1 varieties.

In the present invention and the present description, downy mildew describes a disease caused by a pathogen belonging to the family Peronosporaceae. The main pathogenic organisms responsible for spinach downy mildew are denoted Pfs (Peronospora farinosa f. sp. spinaciae), and currently, races Pfs1 to Pfs19 have been numbered. In addition to these races, many unnumbered races also exist, such as the race that exhibits pathogenicity against the UA1014 strain.

In the present invention and the present description, the terminology "chrX_Y" (wherein X and Y are integers) of a spinach plant means the nucleotide base, within the genome of the spinach plant, which corresponds with base Y of chromosome X (also called the Xth chromosome) in the reference genome (Spinach genome sequence (v1)) published in SpinachBase (http://spinachbase.org/). The "base corresponding with base Y of chromosome X in the SpinachBase reference genome" in any particular spinach plant can be determined by aligning the nucleotide sequence of the genomic DNA of the spinach plant with the nucleotide sequence of the SpinachBase reference genome so as to achieve the highest degree of homology (sequence identity).

Accordingly, the chromosome X of any particular spinach plant can be indicated as the chromosome X of the spinach plant determined by aligning the nucleotide sequence of the genomic DNA of the spinach plant with the nucleotide sequence of the SpinachBase reference genome so as to achieve the highest degree of homology (sequence identity).

In the present invention and the present description, the term "chromosome" includes not only the entire chromosome, but also portions thereof. In other words "a portion of a chromosome" may sometimes simply be referred to as "a chromosome".

An "X gene locus" is a "site occupied by the X gene in the chromosome". Accordingly, in the present invention and the present description, a "spinach plant having an X gene locus" is a spinach plant having a site occupied by the X gene within the chromosome and has the same meaning as a "spinach plant having an X gene".

In the present invention and the present description, a "production method" can also be called a "development method" or a "breeding method". In other words, the terms "production", "development" and "breeding" may all be used with the same meaning.

In the present invention and the present description, the expression "portion of the plant body" includes the cells and tissues of the plant body, and specific examples include the leaves, seeds, flowers, stems, roots, and fruit and the like. In addition, protoplasts obtained from cells of the plant body are also included.

### <Downy Mildew Resistant Spinach Plant>

The downy mildew resistant spinach plant according to the present invention is a spinach plant, other than Spinacia tetrandra, having an RTM-1 gene which is a downy mildew resistance gene located on chromosome 4 of the wild species of spinach Spinacia tetrandra line CGN25466:MGK 01. The RTM-1 gene is located in the range from chr4_7962907 to chr4_8617232 on chromosome 4 of the Spinacia tetrandra line CGN25466:MGK 01.

The RTM-1 gene imparts resistance to at least the downy mildew races Pfs1, Pfs2, Pfs3, Pfs4, Pfs5, Pfs6, Pfs7, Pfs8, Pfs9, Pfs10, Pfs11, Pfs12, Pfs13, Pfs14, Pfs15, Pfs16, Pfs17, Pfs18 and Pfs19, and the race indicated by UA1014 strain. In other words, the "downy mildew resistance derived from the RTM-1 gene" means resistance to at least the downy mildew races Pfs1, Pfs2, Pfs3, Pfs4, Pfs5, Pfs6, Pfs7, Pfs8, Pfs9, Pfs10, Pfs11, Pfs12, Pfs13, Pfs14, Pfs15, Pfs16, Pfs17, Pfs18 and Pfs19, and the race indicated by UA1014 strain. Because this type of resistance to a broad range of races can be imparted with a single gene, the RTM-1 gene is an extremely superior downy mildew resistance gene with resistance not seen conventionally.

The RTM-1 gene is a gene that is dominantly inherited by progeny. As a result, a spinach plant according to the present invention may be a spinach plant in which the RTM-1 gene is homozygous, namely a spinach plant having a homozygous RTM-1 gene, or a spinach plant having a heterozygous RTM-1 gene. Because a spinach plant according to the present invention has the RTM-1 gene, which is a single dominant downy mildew resistance gene which alone is capable of imparting resistance to an extremely broad range of downy mildew races, said spinach plant performs excellent as a downy mildew resistant spinach.

The downy mildew resistant spinach plant according to the present invention can be developed by introducing the RTM-1 gene into the genomic DNA of a spinach plant other than Spinacia tetrandra. This introduction of the RTM-1 gene into the genomic DNA can be achieved, for example, by introducing a chromosome fragment that includes the site where the RTM-1 gene is located (the RTM-1 gene locus) in chromosome 4 of the Spinacia tetrandra line CGN25466:MGK 01. There are no limitations on the site within the chromosome into which the chromosome fragment including the RTM-1 gene locus is introduced, and an extrachromosomal site is also possible. For example, the downy mildew resistant spinach plant according to the present invention includes spinach plants in which a chromosome fragment including the RTM-1 gene locus has been used to substitute a region within chromosome 4 that corresponds with the fragment, as well as spinach plants in which a chromosome fragment including the RTM-1 gene locus has been introduced by translocation or the like into a region other than a region corresponding with the RTM-1 gene locus within chromosome 4 of Spinacia tetrandra.

The chromosome fragment that includes the RTM-1 gene locus may be a fragment, within chromosome 4 of the Spinacia tetrandra line CGN25466:MGK 01, that includes the entire region from chr4_7962907 to chr4_8617232, or may be a partial fragment of the region from chr4_7962907 to chr4_8617232 that includes the RTM-1 gene locus. The partial fragment that includes the RTM-1 gene locus is preferably a fragment that includes the region from chr4_8488603 to chr4_8510715 of chromosome 4 of the Spinacia tetrandra line CGN25466:MGK 01. The above fragment is preferably a chromosome fragment that includes the region from chr4_8488603 to chr4_8510715, from within the region from chr4_7962907 to chr4_8617232. In other words, the downy mildew resistant spinach plant according to the present invention is preferably a spinach plant which, on at least one allele of chromosome 4, contains a fragment that includes the region from chr4_8488603 to chr4_8510715 on chromosome 4 of the Spinacia tetrandra line CGN25466:MGK 01. The chromosome fragment that includes the RTM-1 gene locus may also be a chromosome fragment including the region from chr4_7962907 to chr4_8617232 of chromosome 4 of the Spinacia tetrandra line CGN25466:MGK 01, or a chromosome fragment including the region from chr4_7421480 to chr4_10710358.

Introduction of the RTM-1 gene into the genomic DNA may be conducted by cross breeding methods using Spinacia tetrandra as a parent, or by genetic modification methods such as genome editing methods.

For example, the downy mildew resistant spinach plant according to the present invention may be a plant derived from an interspecific hybrid plant of Spinacia tetrandra and a spinach plant other than Spinacia tetrandra, wherein the plant has downy mildew resistance derived from the RTM-1 gene. The term "interspecific hybrid plant" includes plants produced by crossing of different species of plants belonging to the genus Spinacia, as well as somatic cell hybrid plants produced by interspecific cell fusion of plants of the genus Spinacia, and grafted hybrid plants obtained by interspecific grafting of plants of the genus Spinacia. Further, the expression "interspecific hybrid plant of Spinacia tetrandra and a spinach plant other than Spinacia tetrandra" includes not only the interspecific hybrid plants mentioned above, but also the progeny of those interspecific hybrid plants.

In the present invention and the present description, the expression "progeny of a spinach plant" includes descendants obtained by intraspecific crossing of the spinach plant, as well as individuals obtained with the spinach plant as a parent and the descendants thereof, somatic cell hybrid plants obtained by cell fusion of cells of the spinach plant and cells of a plant of a different variety and the descendants thereof, and individuals obtained by grafting using the spinach plant as either the stock or the scion and the descendants thereof. The term "descendants" incudes both individuals obtained by intraspecific crossing and individuals obtained by interspecific crossing. Further, the expression "individuals obtained with the plant as a parent" means individuals obtained by intraspecific crossing, interspecific crossing, cell fusion or grafting, with the plant acting as a parent.

For example, the downy mildew resistant spinach plant according to the present invention may be developed by crossing Spinacia tetrandra and a spinach plant other than Spinacia tetrandra. In an F1 individual obtained by interspecific crossing with Spinacia tetrandra as a parent, one allele of chromosome 4 is a chromosome 4 having the RTM-1 gene derived from Spinacia tetrandra. Because the RTM-1 gene is a dominant gene, F1 individuals having a chromosome 4 derived from Spinacia tetrandra will have downy mildew resistance derived from the RTM-1 gene.

There are no limitations on the spinach plant used as material in developing the downy mildew resistant spinach plant according to the present invention, and any spinach plant having the RTM-1 gene found in CGN25466:MGK 01 may be used. Examples include Spinacia tetrandra lines other than CGN25466:MGK 01 that are progeny lines that have inherited the RTM-1 gene from CGN25466:MGK 01.

There are no limitations on the parent crossed with Spinacia tetrandra in order to develop the downy mildew resistant spinach plant according to the present invention, provided the parent is a spinach plant other than Spinacia tetrandra, but a cultivated variety of spinach plant that is cultivated as a crop is preferred. Spinacia tetrandra is a wild species, and in terms of use as a crop, characteristics such as the germination properties, pollen quality, leaf color and leaf shape are inferior to the cultivated species of spinach. By using a cultivated variety of spinach as the parent for crossing with Spinacia tetrandra, the characteristics of the resulting downy mildew resistant spinach plant, other than the downy mildew resistance, approach more preferred characteristics for an agricultural crop. Examples of cultivated species of spinach plants include Spinacia oleracea L., and interspecific hybrid spinach plants developed using Spinacia oleracea L. as a parent.

The downy mildew resistant spinach plant according to the present invention may also be a progeny of an F1 individual obtained by interspecific crossing of Spinacia tetrandra and a spinach plant other than Spinacia tetrandra. For example, an F1 seed is obtained by crossing Spinacia tetrandra and a cultivated variety of spinach plant, and a plant obtained by growing the obtained F1 seed is then subjected to either self-pollination or backcrossing with an individual of a cultivated variety of spinach plant to obtain seeds. Subsequently, an individual having the RTM-1 gene is selected from plants obtained by growing the obtained seeds. By repeating the process of subjecting the selected plant individual to self-pollination or crossing with a cultivated variety of spinach plant, and then selecting an individual having the RTM-1 gene from the progeny obtained upon crossing, a phenotypically uniform open pollinated variety of downy mildew resistant spinach plant can be obtained. The self-pollination, crossing with a cultivated variety of spinach plant, cultivation and seed production may be conducted using typical methods employed in spinach cultivation.

Selection of an individual having the RTM-1 gene from progeny obtained by crossing can be achieved, for example, by investigating the downy mildew resistance. Specifically, Pfs of each of the various races may be used to separately inoculate the leaves of a test plant for which downy mildew resistance is to be investigated, and observation then made as to whether or not the disease develops. If downy mildew does not develop, that test plant may be evaluated as having resistance to the various inoculated races. A plant from among the progeny obtained by crossing that exhibits resistance to at least the downy mildew races Pfs1, Pfs2, Pfs3, Pfs4, Pfs5, Pfs6, Pfs7, Pfs8, Pfs9, Pfs10, Pfs11, Pfs12, Pfs13, Pfs14, Pfs15, Pfs16, Pfs17, Pfs18 and Pfs19, and the race differentiated by UA1014 strain may then be selected as an individual having the RTM-1 gene.

Selection of an individual having the RTM-1 gene from progeny obtained by crossing can also be achieved, for example, by using a method for investigating whether the genomic DNA includes a chromosome fragment that includes the RTM-1 gene. Specifically, by using a DNA marker for either the region from chr4_7962907 to chr4_8617232 within chromosome 4 of the spinach plant or a region within the vicinity thereof, an identification can be made as to whether the region is a chromosome fragment derived from the Spinacia tetrandra line CGN25466:MGK 01, or a chromosome fragment derived from a spinach plant other than Spinacia tetrandra.

In the present invention and the present description, a DNA marker is a marker set that can detect differences in DNA sequences on chromosomes to enable discrimination between chromosome fragments derived from different plant species or lines within a species. Examples of DNA markers include SNP (Single Nucleotide Polymorphism, pronounced "snip") markers, SSR (Simple Sequence Repeats) markers and RFLP (Restriction Fragment Length Polymorphism) markers.

DNA marker detection can be achieved using typical methods. For example, using DNA extracted from each plant individual as a template, a nucleic acid amplification reaction may be conducted using a polymerase and a primer capable of hybridizing specifically with a specific SNP or SSR, and an electrophoresis method or the like then used to detect the presence or absence of an amplification product, thus enabling identification of each polymorphism. Alternatively, DNA extracted from each plant individual may be subjected to a restriction enzyme treatment, and an electrophoresis method or the like then used to detect the DNA fragment pattern, thus enabling identification of each polymorphism. Primers capable of hybridizing specifically with a specific SNP or SSR can be designed by typical methods using widely used primer design tools, in accordance with the nucleotide sequence of the genomic DNA of the spinach, and the nucleotide sequence of the SNP or SSR that represents the detection target, and can then be synthesized using methods well known in the technical field.

Examples of DNA markers used in the selection of individuals having the RTM-1 gene include SNPs of the region from chr4_7962907 to chr4_8617232 or regions in the vicinity thereof. Specific examples of these SNPs include chr4_7421480, chr4_7962907, chr4_8152986, chr4_8190990, chr4_8488603, chr4_8494600, chr4_8500200, chr4_8502319, chr4_8502334, chr4_8502394, chr4_8510715, chr4_8617232, and chr4_10710358. Identification of these SNPs can be made, for example, using KASP (Kompetitive Allele Specific PCR) genotyping assay (manufactured by LGC Genomics GmbH). For these SNPs, the genotype of the Spinacia tetrandra line CGN25466:MGK 01 and the genotype of the reference genome published in SpinachBase are shown in Table 1. In Table 1, G represents guanine, C represents cytosine, A represents adenine, and T represents thymine.

**[Table 1]**

| SNP | Spinacia tetrandra genotype | Reference genome genotype |
|---|---|---|
| chr4_7421480 | C | G |
| chr4_7962907 | G | A |
| chr4_8152986 | C | T |
| chr4_8190990 | G | A |
| chr4_8488603 | C | T |
| chr4_8494600 | T | C |
| chr4_8500200 | T | C |
| chr4_8502319 | C | A |
| chr4_8502334 | G | A |
| chr4_8502394 | G | A |
| chr4_8510715 | G | C |
| chr4_8617232 | G | A |
| chr4_10710358 | G | C |

For example, among downy mildew resistant spinach plants according to the present invention, spinach plants having a fragment that includes the range from chr4_8488603 to chr4_8510715 of chromosome 4 of the Spinacia tetrandra line CGN25466:MGK 01 either homozygously or heterozygously have at least one allele of chromosome 4 in which the SNP specified by chr4_8488603 is cytosine, the SNP specified by chr4_8494600 is thymine, and the SNP specified by chr4_8510715 is guanine. Further, for the spinach plant having a fragment that includes the range from chr4_7962907 to chr4_8617232 of chromosome 4 of the Spinacia tetrandra line CGN25466:MGK 01 either homozygously or heterozygously, a spinach plant in which, in at least one allele of chromosome 4, the SNP specified by chr4_7962907 is guanine, the SNP specified by chr4_8152986 is cytosine, the SNP specified by chr4_8190990 is guanine, the SNP specified by chr4_8488603 is cytosine, the SNP specified by chr4_8494600 is thymine, the SNP specified by chr4_8510715 is guanine, and the SNP specified by chr4_8617232 is guanine is preferred. Moreover, for the spinach plant having a fragment that includes the range from chr4_7962907 to chr4_8617232 of chromosome 4 of the Spinacia tetrandra line CGN25466:MGK 01 either homozygously or heterozygously, a spinach plant in which, in at least one allele of chromosome 4, the SNP specified by chr4_7962907 is guanine, the SNP specified by chr4_8152986 is cytosine, the SNP specified by chr4_8190990 is guanine, the SNP specified by chr4_8488603 is cytosine, the SNP specified by chr4_8494600 is thymine, the SNP specified by chr4_8500200 is thymine, the SNP specified by chr4_8502319 is cytosine, the SNP specified by chr4_8502334 is guanine, the SNP specified by chr4_8502394 is guanine, the SNP specified by chr4_8510715 is guanine, and the SNP specified by chr4_8617232 is guanine is also preferred.

The downy mildew resistant spinach plant according to the present invention preferably has characteristics other than the downy mildew resistance, and particularly characteristics making it useful as a crop, that are either the same as, or similar to, cultivated varieties of spinach. Examples of these characteristics include desirable taste, leaf shape, yield, ease of cultivation and the like. The expression "characteristics as a cultivated variety" means that the characteristics making it useful as a crop are characteristics that are suited to use of the plant as an agricultural crop.

Examples of the downy mildew resistant spinach plant according to the present invention include the TNKH-1 line (accession number FERM BP-22404) and the TNKH-2 line (accession number FERM BP-22405). In these two lines, the linkage between the inferior characteristics of Spinacia tetrandra and the RTM-1 gene has been severed, meaning the plants combine the favorable characteristics of a cultivated variety, while retaining the downy mildew resistance derived from the RTM-1 gene. Further, spinach plants having downy mildew resistance derived from the plants specified by these lines are also included within the downy mildew resistant spinach plant according to the present invention.

Examples of spinach plants having downy mildew resistance derived from the plant specified by TNKH-1 include not only the TNKH-1 line itself, but also hybrid plants obtained using the TNKH-1 line as a parent, and the progeny of either of these types of plant, provided the spinach plant has downy mildew resistance derived from the RTM-1 gene. Similar reasoning may also be applied to spinach plants having downy mildew resistance derived from the plant specified by TNKH-2.

The downy mildew resistant spinach plant according to the present invention preferably also has at least one type of downy mildew resistance gene besides the RTM-1 gene. The RTM-1 gene can alone impart resistance to a broader range of races than any known resistance gene, but also incorporating another downy mildew resistance gene can be expected to contribute to suppressing the appearance of new races that may break through this resistance. Although there are no limitations on the downy mildew resistance gene besides the RTM-1 gene, examples include the RPF1 to RPF15 genes, the R6 gene, and the R15 gene and the like.

The downy mildew resistant spinach plant according to the present invention may be either the entirety or a portion of the plant body. In other words, the downy mildew resistant spinach plant according to the present invention includes the entire plant body of the spinach plant having the RTM-1 gene, the above ground portion of the spinach plant, and the tissues of the spinach plant. Further, the downy mildew resistant spinach plant according to the present invention also includes cells obtained from any tissue of the spinach plant having the RTM-1 gene. Examples of this tissue include the embryo, meristem cells, callus, pollen, leaves, anthers, stem, petioles, roots, root apices, fruit, seeds, flowers, cotyledons, and hypocotyl and the like.

The downy mildew resistance of the downy mildew resistant spinach plant according to the present invention exhibits dominant expression. As a result, by conducting interspecific crossing or intraspecific crossing using the downy mildew resistant spinach plant according to the present invention as a parent, novel lines of spinach plants having downy mildew resistance derived from the RTM-1 gene can be developed.

### <Method for Screening Downy Mildew Resistant Spinach Plants>

A method for screening downy mildew resistant spinach plants according to the present invention includes investigating whether a test spinach plant has the RTM-1 gene, and then selecting the spinach plant as a downy mildew resistant spinach plant in those cases where the test plant is determined as having the RTM-1 gene. In the method for screening downy mildew resistant spinach plants according to the present invention, the determination as to whether the test spinach plant has the RTM-1 gene can be made on the basis of the genotype of a DNA marker for the RTM-1 gene locus of chromosome 4 or a region within the vicinity thereof, or the genotype of a DNA marker strongly linked with these DNA markers. In those cases where, in at least one allele of chromosome 4, the genotype of a DNA marker for the RTM-1 gene locus of chromosome 4 or a region within the vicinity thereof or a DNA marker strongly linked with these DNA markers has the same genotype as that of a Spinacia tetrandra such as CGN25466:MGK 01 which has an RTM-1 gene and exhibits downy mildew resistance, the test spinach plant may be selected as a downy mildew resistant spinach plant.

Examples of DNA markers that can be used for determining the presence or absence of the RTM-1 gene include the SNPs shown in Table 1. Among these, at least one SNP selected from the group consisting of the SNP specified by chr4_8488603, the SNP specified by chr4_8494600, the SNP specified by chr4_8510715, and SNPs strongly linked to these SNPs, is ideal as the DNA marker for determining the presence or absence of the RTM-1 gene. For example, in those cases where, in at least one allele, the SNP specified by chr4_8488603 is cytosine, the SNP specified by chr4_8494600 is thymine, or the SNP specified by chr4_8510715 is guanine, the spinach plant may be selected as a downy mildew resistant spinach plant.

By preparing a kit form of a primer set for identifying the genotype of a DNA marker used for determining the presence or absence of the RTM-1 gene, and then using that kit, the determination of the presence or absence of the RTM-1 gene, and subsequent selection of downy mildew resistant spinach plants can be performed more easily. For example, a kit that combines at least one primer set selected from the group consisting of a primer set for identifying the genotype of the SNP specified by chr4_8488603, a primer set for identifying the genotype of the SNP specified by chr4_8494600, and a primer set for identifying the genotype of the SNP specified by chr4_8510715 is useful in identifying the genotypes of these SNPs used in determining the presence or absence of the RTM-1 gene in the test spinach plant.

### <Method for Predicting Downy Mildew Resistance of Spinach Plants>

A method for predicting the downy mildew resistance of a spinach plant according to the present invention includes investigating whether a test spinach plant has the RTM-1 gene, and in those cases where the test plant is determined as having the RTM-1 gene, predicting that the test spinach plant will have a high probability of exhibiting downy mildew resistance. In the method for predicting the downy mildew resistance of a spinach plant according to the present invention, the determination as to whether the test spinach plant has the RTM-1 gene can be made on the basis of the genotype of a DNA marker for the RTM-1 gene locus of chromosome 4 or a region within the vicinity thereof, or the genotype of a DNA marker strongly linked with these DNA markers. In those cases where, in at least one allele of chromosome 4, the genotype of a DNA marker for the RTM-1 gene locus of chromosome 4 or a region within the vicinity thereof or a DNA marker strongly linked with these DNA markers has the same genotype as that of a Spinacia tetrandra such as CGN25466:MGK 01 which has an RTM-1 gene and exhibits downy mildew resistance, the spinach plant may be determined as having the RTM-1 gene, whereas in those cases where the genotype differs from that of a Spinacia tetrandra such as CGN25466:MGK 01 which has an RTM-1 gene and exhibits downy mildew resistance, the spinach plant may be determined as lacking the RTM-1 gene.

Examples of DNA markers that can be used for determining the presence or absence of the RTM-1 gene include the SNPs shown in Table 1. Among these, at least one SNP selected from the group consisting of the SNP specified by chr4_8488603 of the spinach plant, the SNP specified by chr4_8494600, the SNP specified by chr4_8510715, and SNPs genetically strongly linked to these SNPs is ideal as the DNA marker for predicting downy mildew resistance. For example, in those cases where, in at least one allele, the SNP specified by chr4_8488603 is cytosine, the SNP specified by chr4_8494600 is thymine, or the SNP specified by chr4_8510715 is guanine, the test spinach plant may be predicted as having the RTM-1 gene and having a high probability of exhibiting downy mildew resistance. Primer sets and kits for identifying the genotypes of DNA markers used in determining the presence or absence of the RTM-1 gene may employ the same primer sets and kits as those described above.

### <Method for Producing Downy Mildew Resistant Spinach Plant>

By conducting cross-pollination using a spinach plant having the RTM-1 gene as a donor material, downy mildew resistance derived from the RTM-1 gene can be introduced into a spinach plant not having the RTM-1 gene, thus producing a novel downy mildew resistant spinach plant. The RTM-1 gene, which is a single dominant resistance gene, can be easily introduced into an arbitrary line, meaning the breeding of spinach varieties can be accelerated. Moreover, the RTM-1 gene can be combined with another single dominant resistant allele in the same haplotype. Accordingly, by using a spinach plant having the RTM-1 gene as a parent, varieties having resistance to a broader range of races can be developed.

The method for producing a downy mildew resistant spinach plant according to the present invention is a method that utilizes cross breeding using a spinach plant having the RTM-1 gene as a parent. Specifically, the method has a first crossing step of crossing a spinach plant having the RTM-1 gene and an arbitrary spinach plant, a second crossing step of obtaining a segregating population by subjecting an F1 individual obtained in the first crossing step to self-pollination, backcrossing, or interspecific crossing or intraspecific crossing with a spinach plant different from the parent used in the first crossing step, and a selection step of selecting a spinach plant having the RTM-1 gene from the segregating population. The self-pollination, backcrossing, interspecific crossing, intraspecific crossing, spinach plant cultivation and seed production may be conducted using typical methods employed in spinach cultivation.

Spinacia tetrandra may be used as the spinach plant having the RTM-1 gene used as a parent in the first crossing step, and the downy mildew resistant spinach plant of the present invention described above may also be used. In the present invention, the spinach plant having the RTM-1 gene used as a parent is preferably a downy mildew resistant spinach plant that has the RTM-1 gene, and combines the characteristics of a cultivated variety. Examples of such downy mildew resistant spinach plants include the TNKH-1 line, hybrid plants obtained using the TNKH-1 line as a parent, the TNKH-2 line, hybrid plants obtained using the TNKH-2 line as a parent, and the progeny of any of these plants, wherein the spinach plant has downy mildew resistance derived from the RTM-1 gene.

There are no limitations on the arbitrary spinach plant used as a parent in the first crossing step, or the arbitrary spinach plant used as a parent for the interspecific crossing or intraspecific crossing in the second crossing step, and any appropriate type of spinach plant lacking the RTM-1 gene may be used. In the present invention, the use of a cultivated variety of spinach plant as this parent is preferred. For example, by using a downy mildew resistant spinach plant having the RTM-1 gene while also combining the characteristics of a cultivated variety, and a cultivated variety of spinach plant lacking the RTM-1 gene as parents, conducting crossing of these two parents, and then subjecting an obtained F1 individual to self-pollination, backcrossing, or interspecific crossing or intraspecific crossing with a cultivated variety of spinach plant different from the parent used in the first crossing step, a novel spinach line having downy mildew resistance derived from the RTM-1 gene as well as the favorable characteristics of a cultivated variety can be developed comparatively easily.

The arbitrary spinach plant used as a parent in the first crossing step, and the arbitrary spinach plant used as a parent for the interspecific crossing or intraspecific crossing in the second crossing step are preferably spinach plants having at least one type of downy mildew resistance gene other than the RTM-1 gene, and are more preferably cultivated varieties of spinach plants having at least one type of downy mildew resistance gene other than the RTM-1 gene. By using a spinach plant having at least one type of downy mildew resistance gene other than the RTM-1 gene as a parent, a novel spinach plant including both the RTM-1 gene and another downy mildew resistance gene can be developed comparatively easily.

In the selection step, the selection of a spinach plant having the RTM-1 gene from the segregating population may be conducted, for example, by inoculating the leaves of a test plant separately with Pfs of each of the various races and investigating the downy mildew resistance. A plant from within the segregating population that exhibits resistance to at least the downy mildew races Pfs1, Pfs2, Pfs3, Pfs4, Pfs5, Pfs6, Pfs7, Pfs8, Pfs9, Pfs10, Pfs11, Pfs12, Pfs13, Pfs14, Pfs15, Pfs16, Pfs17, Pfs18 and Pfs19, and the race indicated by UA1014 strain is then selected as an individual having the RTM-1 gene.

Selection of an individual having the RTM-1 gene in the selection step may be conducted using a DNA marker for the RTM-1 gene locus in chromosome 4 or a region in the vicinity thereof. The DNA marker and the specific method used may be the same as those described above in relation to the screening method according to the present invention.

In those cases where a plant individual including both the RTM-1 gene and another downy mildew resistance gene is to be selected, a DNA marker capable of identifying the presence or absence of the other downy mildew resistance gene is used together with the DNA marker described above for selection of an individual having the RTM-1 gene. Examples of DNA markers that may be used for identifying the presence or absence of the other downy mildew resistance gene include SNPs that exist in the gene locus of the other downy mildew resistance gene or in the vicinity thereof.

Subsequently, by repeating self-pollination of seeds produced from a selected progeny individual, a downy mildew resistant spinach plant can be obtained which exhibits more stable characteristics, and a more stable germination rate, yield, and seed productivity and the like that enable cultivation as a crop. There are no limitations on the number of repetitions of the self-pollination process provided the number is one or more, but the number of repetitions may be 2 or 3, or greater than 3.

### [Examples]

Next, the present invention is described in further detail using a series of examples, but the present invention is not limited to the following examples.

### <Race Inoculation Test for Investigating Downy Mildew Resistance>

Pfs inoculation tests on spinach plants were conducted using the method described below.

Spinach seeds were sown in a tray filled with seedling soil "Super Mix A" (manufactured by Sakata Seed Corporation), and then covered with another seedling soil "Metro-Mix 350" (manufactured by Hyponex Japan Co., Ltd.). During sowing, seeds of a line that exhibits susceptibility to the inoculated race were also sown in each tray to provide a control group. The tray was placed in a glasshouse for two weeks to raise the seedlings, and at the two-leaf stage, the seedlings were inoculated by spraying with a Pfs spore suspension (5×10⁴ /mL). Following inoculation, the tray was immediately covered with a plastic cover, and maintained under conditions including a temperature of 15°C, humidity of 100% and 12-hour long days.

Seven to ten days after inoculation, once disease was obvious in the control group, each individual seedling in the test population was evaluated for resistance or susceptibility. Plants for which mycelia and spores had appeared on the cotyledons or true leaves, confirming disease, were evaluated as being susceptible to disease, whereas plants for which no disease could be confirmed on either the cotyledons or the true leaves were deemed resistant.

Each race of downy mildew was acquired from within Japan, from The University of Arkansas (Dr. Jim Correll) or from Naktuinbouw. Furthermore, differentiation of each downy mildew race was conducted using known spinach varieties that exhibit different resistance patterns to the various races as differential sets. These differential sets used in differentiating resistance to each downy mildew race can be acquired from USDA (United States Department of Agriculture) and Naktuinbouw (Non-Patent Document 4).

The resistances reported for each of the various differential sets are shown in Table 2 and Table 3. In the tables, "-" means resistance, "(-)" means medium resistance, "+" means disease susceptibility, and "(+)" means that disease symptoms and spore formation were observed only on the cotyledons, with no symptoms observed on the true leaves. Furthermore, "*" means that the resistance results differed for each test.

**[Table 2]**

| Differential | | Viroflay | NIL 5 | NIL 3 | NIL 4 | NIL 6 | NIL 1 |
|---|---|---|---|---|---|---|---|
| Race - Pfs: | 1 | + | - | - | - | - | - |
| | 2 | + | - | + | - | + | - |
| | 3 | + | + | - | - | - | - |
| | 4 | + | + | + | - | - | - |
| | 5 | + | + | - | + | - | - |
| | 6 | + | + | + | + | + | - |
| | 7 | + | + | + | + | - | - |
| | 8 | + | + | - | + | + | + |
| | 9 | + | + | - | + | + | - |
| | 10 | + | + | + | + | + | + |
| | 11 | + | + | - | + | - | - |
| | 12 | + | + | - | + | + | + |
| | 13 | + | + | + | + | - | (-) |
| | 14 | + | + | - | + | + | + |
| | 15 | + | + | + | - | - | - |
| | 16 | + | + | - | + | - | - |
| | 17 | + | + | + | + | + | + |
| | 18 | + | + | + | + | - | - |
| | 19 | + | + | - | + | + | + |
| | UA1014 strain | + | + | + | + | + | + |

**[Table 3]**

| Differential | | NIL 2 | Pigeon | Calandonia | Meerkat | Hydrus |
|---|---|---|---|---|---|---|
| Race - Pfs: | 1 | - | - | - | - | - |
| | 2 | - | - | - | - | - |
| | 3 | - | - | - | - | - |
| | 4 | - | - | - | - | - |
| | 5 | - | - | - | - | - |
| | 6 | - | - | - | - | - |
| | 7 | - | - | - | - | - |
| | 8 | - | - | - | - | - |
| | 9 | - | - | - | - | - |
| | 10 | - | - | - | - | - |
| | 11 | + | - | - | - | - |
| | 12 | + | - | - | - | - |
| | 13 | + | - | - | - | - |
| | 14 | + | + | - | - | - |
| | 15 | - | - | + | - | - |
| | 16 | + | + | - | + | - |
| | 17 | + | + | + | (-) | - |
| | 18 | + | + | + | + | - |
| | 19 | + | + | - | + | + |
| | UA1014 strain | + | (+) | (+) | (+) | -/(+)* |

### [Example 1]

Using the wild species Spinacia tetrandra line CGN25466:MGK 01 acquired from CGN of Holland in 2013 as a parent, a novel downy mildew resistant line was developed.

First, of the Spinacia tetrandra lines acquired from CGN of Holland, CGN25466:MGK 01 and CGN25474:MGK 18 were sown, and the germinated individuals were inoculated with Pfs6. The results revealed that whereas Spinacia tetrandra line CGN25466:MGK 01 exhibited resistance, Spinacia tetrandra line CGN25474:MGK 18 exhibited disease susceptibility. Accordingly, the decision was made to use Spinacia tetrandra line CGN25466:MGK 01 as a parent.

First, 60 seeds of Spinacia tetrandra line CGN25466:MGK 01 were sown, and 25 of the germinated individuals were inoculated with Pfs10. The results revealed that all 25 individuals exhibited resistance.

Subsequently, four individuals from these 25 individuals were crossed with an advanced parent line having the RPF3 gene, and F1 seeds were obtained. An advanced parent line means a parent that is desirable as a parent for developing a line that exhibits favorable characteristics as a cultivated variety.

When 40 of the obtained F1 seeds were sown, 10 individual plants grew. Two individuals among these 10 individuals were subjected to backcrossing with the above advanced line to obtain BC1 (backcross 1) generation seeds.

When 24 of the obtained BC1 seeds were sown, 10 individual plants grew. When these 10 individuals were inoculated with Pfs10, to which RPF3 exhibits no resistance, 7 of the individuals exhibited resistance. One of those individuals was crossed with an advanced parent line (having the RPF4 gene), and BC1F1 generation seeds were obtained.

When 50 of these BC1F1 seeds were sown, 43 individual plants grew. When these 43 individuals were inoculated with Pfs10, to which neither RPF3 nor RPF4 exhibits resistance, 15 of the individuals exhibited resistance. Of those 15 individuals, 11 individuals were subjected to self-pollination, and BC1F1S1 generation seeds were obtained.

When 817 of the obtained BC1F1S1 seeds were sown, 676 individual plants grew. All of the grown plants were inoculated with Pfs10, and 500 individual plants exhibited resistance. Of the individuals that exhibited resistance, 22 individuals were subjected to self-pollination, and BC1F1S2 generation seeds were obtained.

When 7,696 of the obtained BC1F1S2 seeds were sown, 3,551 individual plants grew. Of these plants, 76 individuals were subjected to self-pollination, and BC1F1S3 generation seeds were obtained.

When 12,971 of the obtained BC1F1S3 seeds were sown, 9,815 individual plants grew. Of these plants, 115 individuals were subjected to self-pollination, and BC1F1S4 generation seeds were obtained.

When 7,377 of the BC1F1S4 seeds from theses 115 self-pollinations were sown, 4,717 individual plants grew. When these plants were inoculated with Pfs10 or the race indicated by the UA1014 strain, to which neither RPF3 nor RPF4 exhibits resistance, 100% of the plants from 5 of the 115 harvests exhibited resistance to both Pfs10 and the race differentiated by the UA1014 strain. It was concluded that these 5 lines possess a previously unknown homozygous downy mildew resistance gene different from RPF3 and RPF4, and this unknown downy mildew resistance gene is not an allele of the RPF3 gene or RPF4 gene, but rather it exists at a different gene locus. Next, 34 individuals of the 5 lines that exhibited 100% resistance were subjected to self-pollination, and BC1F1S5 generation seeds were obtained.

For the 5 lines for which all of the individuals exhibited resistance, the presence or absence of the RPF3 gene and RPF4 gene was investigated using an RPF3 marker and an RPF4 marker. Based on the results, an RPF3 homozygous line was selected and named the TNKH-1 line. Further, an RPF4 homozygous line was selected and named the TNKH-2 line. Moreover, a segregating line of RPF3 and RPF4 was selected and named the TNKH-3 line. The unknown downy mildew resistance gene and gene locus within these lines, derived from the Spinacia tetrandra, was named RTM-1.

In the Spinacia tetrandra line and the early generations in which the genetic effects of the Spinacia tetrandra were strongest, pluralities of seeds tended to clump together, and germination was also poor. Accordingly, harvesting and cleaning of the seeds required considerable labor, and to improve the germination rate, considerable attention was paid to the germination environment following seed sowing. Furthermore, the pollen from these plants was heavy and difficult to disperse, meaning cross-pollination was difficult. Moreover, in each step, selection was conducted not only based on disease resistance, but also with due consideration of other factors such as the leaf color, leaf shape and leaf size, and as far as possible, those individuals having characteristics close to cultivated species were selected.

For the TNKH-1 line and the TNKH-2 line, the applicants deposited seeds of these lines in the National Institute of Technology and Evaluation (NITE) International Patent Organism Depositary (Room 120, 2-5-8 Kazusakamatari, Kisarazu-shi, Chiba, Japan). The identification code appended to the TNKH-1 line by the depositor is SSC-SPI-20-001, and the accession number is FERM BP-22404 (accession date: 25 December 2020), whereas the identification code appended to the TNKH-2 line by the depositor is SSC-SPI-20-002, and the accession number is FERM BP-22405 (accession date: 25 December 2020).

### [Example 2]

Cross-pollination was conducted using the Viroflay spinach variety, which lacks resistance to any race of downy mildew, as the seed parent, and the TNKH-3 line developed in Example 1 as the pollen parent, the thus obtained F1 individuals were subjected to inoculation tests using Pfs1, Pfs2, Pfs3, Pfs4, Pfs5, Pfs6, Pfs7, Pfs8, Pfs10, Pfs11, Pfs12, Pfs13, Pfs14, Pfs15, Pfs16, Pfs17 and the race indicated by UA1014 strain, and the resistance to these races was investigated. The results are shown in Table 4. In Table 4, in the column labeled "Viroflay × TNKH-3 F1", the columns displaying numbers of individuals for "resistant individuals" and "susceptible individuals" uses a format P(Q) (where P and Q are both integers), wherein P indicates the number of F1 individuals among the resistant individuals or susceptible individuals, and Q indicates the number of individuals obtained by Viroflay self-pollination among the resistant individuals or susceptible individuals. Further, "nt" means that no test was conducted, and no data is available. Moreover, within the columns labeled "RPF3 line", RPF4 line" and "Viroflay", in the table cell for each race, "-" indicates resistance and "+" indicates disease susceptibility. Of the RTM-1 genotypes shown in Table 4, RTM-1 means a resistant genotype derived from Spinacia tetrandra, and rtm-1 means a susceptible genotype derived from Viroflay. Accordingly, RTM-1/RTM-1 means a resistant homozygote, rtm-1/rtm-1 means a susceptible homozygote, and RTM-1/rtm-1 means a resistant heterozygote. Similarly, for the RPF genotypes, rpfV means a susceptible genotype derived from Viroflay.

**[Table 4]**

| Line | | TNKH-3 | | Viroflay × TNKH-3 F1 | | RPF3 line | RPF4 line | Viroflay |
|---|---|---|---|---|---|---|---|---|
| RTM-1 genotype | | RTM-1/RTM-1 | | RTM-1/rtm-1 | | rtm-1/ rtm-1 | rtm-1/ rtm-1 | rtm-1/ rtm-1 |
| RPF genotype | | Segregation | | | | RPF3/ RPF3 | RPF4/ RPF4 | rpfV/ rpfV |
| | | RPF3/RPF3 | | RPF3/rpfV | | | | |
| | | RPF3/RPF4 | | RPF4/rpfV | | | | |
| | | RPF4/RPF4 | | | | | | |

| Number of individuals | | Resistant individuals | Susceptible individuals | Resistant individuals | Susceptible individuals | | | |
|---|---|---|---|---|---|---|---|---|
| Race - Pfs: | 1 | 60 | 0 | 9(0) | 0(0) | - | - | + |
| | 2 | 14 | 0 | 21(0) | 0(1) | + | - | + |
| | 3 | 17 | 0 | 17(0) | 0(2) | - | - | + |
| | 4 | 11 | 0 | 9(0) | 0(4) | + | - | + |
| | 5 | 14 | 0 | 21(0) | 0(1) | - | + | + |
| | 6 | 24 | 0 | 14(0) | 0(5) | + | + | + |
| | 7 | 17 | 0 | 14(0) | 0(2) | + | + | + |
| | 8 | 17 | 0 | 14(0) | 0(2) | - | + | + |
| | 9 | nt | nt | nt | nt | - | + | + |
| | 10 | 24 | 0 | 12(0) | 0(5) | + | + | + |
| | 11 | 18 | 0 | 16(0) | 0(4) | - | + | + |
| | 12 | 24 | 0 | 13(0) | 0(2) | - | + | + |
| | 13 | 10 | 0 | 15(0) | 0(0) | + | + | + |
| | 14 | 18 | 0 | 14(0) | 0(3) | - | + | + |
| | 15 | 17 | 0 | 17(0) | 0(2) | + | - | + |
| | 16 | 19 | 0 | 14(0) | 0(6) | - | + | + |
| | 17 | 22 | 0 | 14(0) | 0(1) | + | + | + |
| | UA1014 strain | 27 | 0 | 14(0) | 0(1) | + | + | + |

Spinach is a dioecious species, but lines also exist which are monoecious, in which both pistillate and staminate flowers develop within the same individual. In the production of F1 seeds using Viroflay as the seed parent, seeds resulting from Viroflay self-pollination are sometimes also included in the harvest. Whether a seed is a Viroflay self-pollination seed or an F1 seed can be determined by investigating the RPF genotype using an RPF3 marker and an RPF4 marker.

In the F1 population obtained by crossing Viroflay and the TNKH-3 line, when the RPF genotype of the susceptible individuals was investigated, it was found that the individuals lacked both the RPF3 gene and the RPF4 gene (rpfV/rpfV), confirming that all of these susceptible individuals were obtained by Viroflay self-pollination. In other words, as illustrated in Table 4, the F1 individuals obtained by crossing the Viroflay line and the TNKH-3 line exhibited resistance to all races except for Pfs9, and no susceptible individuals were produced. Based on the downy mildew resistance of the RPF3 gene and the RPF4 gene, it was clear that the RTM-1 gene was capable of imparting dominant resistance to at least Pfs6, Pfs7, Pfs10, Pfs13, Pfs17, and the race indicated by UA1014 strain.

In the F1 population, RPF3/rpfV and RPF4/rpfV segregated approximately 1: 1. RPF3 is susceptible to Pfs2, Pfs4 and Pfs15, and RPF4 is susceptible to Pfs5, Pfs8, Pfs11, Pfs12, Pfs14 and Pfs16, and therefore if the assumption were made that the RTM-1 gene had no resistance to these races, then for these races, the numbers of individuals in the ratio resistant individuals : susceptible individuals should yield an expected value of 1:1. However, the actual results revealed that all of the F1 individuals exhibited resistance to these races. Based on these results, it is clear that the RTM-1 gene is also capable of imparting dominant resistance to Pfs2, Pfs4, Pfs5, Pfs8, Pfs11, Pfs12, Pfs14, Pfs15 and Pfs16.

### [Example 3]

Cross-pollination was conducted using Viroflay as the seed parent, and the TNKH-1 line developed in Example 1 as the pollen parent. The thus obtained F1 individuals were subjected to inoculation tests using Pfs2, Pfs4, Pfs10, Pfs15, Pfs17, Pfs18 and the race indicated by UA1014 strain, and the resistance to these races was investigated. The results are shown in Table 5. In Table 5, in the column labeled "Viroflay × TNKH-1 F1", the columns displaying numbers of individuals for "resistant individuals" and "susceptible individuals" use a format P(Q) (wherein P and Q are both integers), wherein P indicates the number of F1 individuals among the resistant individuals or susceptible individuals, and Q indicates the number of individuals obtained by Viroflay self-pollination among the resistant individuals or susceptible individuals. Further, "nt" means that no test was conducted, and no data is available. Moreover, within the columns labeled "RPF3 line" and "Viroflay", in the table cell for each race, "-" indicates resistance and "+" indicates disease susceptibility. Of the RTM-1 genotypes shown in Table 5, RTM-1 means a resistant genotype derived from Spinacia tetrandra, and rtm-1 means a susceptible genotype derived from Viroflay. Accordingly, RTM-1/RTM-1 means a resistant homozygote, rtm-1/rtm-1 means a susceptible homozygote, and RTM-1/rtm-1 means a resistant heterozygote. Similarly, for the RPF genotypes, rpfV means a susceptible genotype derived from Viroflay.

**[Table 5]**

| Line | | TNKH-1 | | Viroflay × TNKH-1 F1 | | RPF3 line | Viroflay |
|---|---|---|---|---|---|---|---|
| RTM-1 genotype | | RTM-1/RTM-1 | | RTM-1/rtm-1 | | rtm-1/ rtm-1 | rtm-1/ rtm-1 |
| RPF genotype | | RPF3/RPF3 | | RPF3/rpfV | | RPF3/RPF3 | rpfV/rpfV |

| Number of individuals | | Resistant individuals | Susceptible individuals | Resistant individuals | Susceptible individuals | | |
|---|---|---|---|---|---|---|---|
| Race - Pfs: | 2 | 29 | 0 | 30(0) | 0(6) | + | + |
| | 4 | 20 | 0 | 27(0) | 0(2) | + | + |
| | 10 | 26 | 0 | 34(0) | 0(2) | + | + |
| | 15 | 20 | 0 | 15(0) | 0(3) | + | + |
| | 17 | 17 | 0 | 19(0) | 0(0) | + | + |
| | 18 | 35 | 0 | 14(0) | 0(2) | + | + |
| | UA1014 strain | 30 | 0 | 34(0) | 0(1) | + | + |

As illustrated in Table 5, the F1 individuals obtained by crossing the Viroflay line and the TNKH-1 line exhibited resistance to Pfs2, Pfs4, Pfs10, Pfs15, Pfs17, Pfs18 and the race indicated by UA1014 strain, and no susceptible individuals were observed. The results of investigating the RPF genotypes confirmed that all of the susceptible individuals were individuals obtained by Viroflay self-pollination. Based on the downy mildew resistance of the RPF3 gene, it was clear that the RTM-1 gene was capable of imparting dominant resistance to at least Pfs2, Pfs4, Pfs10, Pfs15, Pfs17, Pfs18, and the race indicated by UA1014 strain.

### [Example 4]

Cross-pollination was conducted using Viroflay as the seed parent, and the TNKH-2 line developed in Example 1 as the pollen parent, the thus obtained F1 individuals were subjected to inoculation tests using Pfs5, Pfs8, Pfs9, Pfs10, Pfs11, Pfs12, Pfs14, Pfs16, Pfs17, Pfs18, Pfs19 and the race indicated by UA1014 strain, and the resistance to these races was investigated. The results are shown in Table 6. In Table 6, in the column labeled "Viroflay × TNKH-2 F1", the columns displaying numbers of individuals for "resistant individuals" and "susceptible individuals" use a format P(Q) (wherein P and Q are both integers), wherein P indicates the number of F1 individuals among the resistant individuals or susceptible individuals, and Q indicates the number of individuals obtained by Viroflay self-pollination among the resistant individuals or susceptible individuals. Further, "nt" means that no test was conducted, and no data is available. Moreover, within the columns labeled "RPF4 line" and "Viroflay", in the table cell for each race, "-" indicates resistance and "+" indicates disease susceptibility. Of the RTM-1 genotypes shown in Table 6, RTM-1 means a resistant genotype derived from Spinacia tetrandra, and rtm-1 means a susceptible genotype derived from Viroflay. Accordingly, RTM-1/RTM-1 means a resistant homozygote, rtm-1/rtm-1 means a susceptible homozygote, and RTM-1/rtm-1 means a resistant heterozygote. Similarly, for the RPF genotypes, rpfV means a susceptible genotype derived from Viroflay.

**[Table 6]**

| Line | | TNKH-2 | | Viroflay × TNKH-2 F1 | | RPF4 line | Viroflay |
|---|---|---|---|---|---|---|---|
| RTM-1 genotype | | RTM-1/RTM-1 | | RTM-1/rtm-1 | | rtm-1/ rtm-1 | rtm-1/ rtm-1 |
| RPF genotype | | RPF4/RPF4 | | RPF4/rpfV | | RPF4/RPF4 | rpf V/rpf V |

| Number of individuals | | Resistant individuals | Susceptible individuals | Resistant individuals | Susceptible individuals | | |
|---|---|---|---|---|---|---|---|
| Race - Pfs: | 5 | 18 | 0 | 14(0) | 0(3) | + | + |
| | 8 | 24 | 0 | 12(0) | 0(3) | + | + |
| | 9 | 21 | 0 | 19(0) | 0(8) | + | + |
| | 10 | 24 | 0 | 14(0) | 0(7) | + | + |
| | 11 | 14 | 0 | 1(0) | 0(2) | + | + |
| | 12 | 25 | 0 | 14(0) | 0(6) | + | + |
| | 14 | 27 | 0 | 17(0) | 0(8) | + | + |
| | 16 | 24 | 0 | 25(0) | 0(6) | + | + |
| | 17 | 16 | 0 | 18(0) | 0(0) | + | + |
| | 18 | 20 | 0 | 11(0) | 0(16) | + | + |
| | 19 | 29 | 0 | 24(0) | 0(13) | + | + |
| | UA1014 strain | 29 | 0 | 10(0) | 0(11) | + | + |

As illustrated in Table 6, the F1 individuals obtained by crossing the Viroflay line and the TNKH-2 line exhibited resistance to Pfs5, Pfs8, Pfs9, Pfs10, Pfs11, Pfs12, Pfs14, Pfs16, Pfs17, Pfs18, Pfs19 and the race indicated by UA1014 strain, and no susceptible individuals were observed. The results of investigating the RPF genotypes confirmed that all of the susceptible individuals were individuals obtained by Viroflay self-pollination. Based on the downy mildew resistance of the RPF4 gene, it was clear that the RTM-1 gene was capable of imparting dominant resistance to at least Pfs5, Pfs8, Pfs9, Pfs10, Pfs11, Pfs12, Pfs14, Pfs16, Pfs17, Pfs18, Pfs19 and the race indicated by UA1014 strain.

### [Example 5]

Cross-pollination was conducted using Viroflay as the seed parent, and the TNKH-1 line developed in Example 1 as the pollen parent. The thus obtained F1 seeds were self-pollinated to produce an F2 segregating population. This F2 population was subjected to inoculation tests using Pfs1, Pfs3, Pfs4, Pfs8, Pfs10, Pfs11, Pfs12, Pfs14, Pfs15 and the race indicated by UA1014 strain, and the resistance to these races was investigated. The results are shown in Table 7. In Table 7, "nt" means that no test was conducted, and no data is available. Moreover, within the columns labeled "RPF3" and "Viroflay", in the table cell for each race, "-" indicates resistance and "+" indicates disease susceptibility. Of the RTM-1 genotypes shown in Table 7, RTM-1 means a resistant genotype derived from Spinacia tetrandra, and rtm-1 means a susceptible genotype derived from Viroflay. Accordingly, RTM-1/RTM-1 means a resistant homozygote, rtm-1/rtm-1 means a susceptible homozygote, and RTM-1/rtm-1 means a resistant heterozygote. Similarly, for the RPF genotypes, rpfV means a susceptible genotype derived from Viroflay.

**[Table 7]**

| Line | | Viroflay × TNKH-1 F2 | | | | RPF3 | Viroflay |
|---|---|---|---|---|---|---|---|
| RTM-1 genotype | | Segregation | | Assumed mode of inheritance | χ2 test (p>0.05) | rtm-1/ rtm-1 | rtm-1/ rtm-1 |
| | | RTM-1/RTM-1 | | | | | |
| | | RTM-1/rtm-1 | | | | | |
| | | rtm-1/rtm-1 | | | | | |
| RPF genotype | | Segregation | | | | RPF3/ RPF3 | rpfV/ rpfV |
| | | RPF3/RPF3 | | | | | |
| | | RPF3/rpfV | | | | | |
| | | rpfV/rpfV | | | | | |

| Number of individuals | | Resistant individuals | Susceptible individuals | | | | |
|---|---|---|---|---|---|---|---|
| Race - Pfs: | 1 | 76 | 8 | Two-factor dominant | 0.215 | - | + |
| | 3 | 106 | 8 | Two-factor dominant | 0.735 | - | + |
| | 4 | 63 | 19 | Single factor dominant | 0.702 | + | + |
| | 8 | 76 | 6 | Two-factor dominant | 0.690 | - | + |
| | 10 | 57 | 23 | Single factor dominant | 0.439 | + | + |
| | 11 | 69 | 8 | Two-factor dominant | 0.133 | - | + |
| | 12 | 76 | 6 | Two-factor dominant | 0.690 | - | + |
| | 14 | 306 | 27 | Two-factor dominant | 0.161 | - | + |
| | 15 | 59 | 21 | Single factor dominant | 0.796 | + | + |
| | UA1014 strain | 237 | 98 | Single factor dominant | 0.072 | + | + |

As illustrated in Table 7, the downy mildew resistance of the TNKH-1 line to Pfs4, Pfs10, Pfs15 and the race indicated by UA1014 strain, all of which are races to which the RPF3 gene is susceptible, segregated into a ratio of resistant individuals : susceptible individuals of close to 3: 1. Accordingly, RTM-1 was assumed to be a single dominant gene. This assumption was also supported by a χ² test (p>0.05).

The downy mildew resistance of the TNKH-1 line to Pfs1, Pfs3, Pfs8, Pfs11, Pfs12 and Pfs14, all of which are races to which the RPF3 gene is resistant, segregated into a ratio of resistant individuals : susceptible individuals of close to 15: 1. Accordingly, the RTM-1 gene and the RPF3 gene were assumed to be two independent dominant genes. This assumption was also supported by a χ² test (p>0.05).

Based on these results, it was evident that the RTM-1 gene is not an allele of the RPF gene, is located on a different chromosome, and is capable of imparting single dominant resistance to Pfs1, Pfs3, Pfs4, Pfs8, Pfs10, Pfs11, Pfs12, Pfs14, Pfs15, and the race indicated by UA1014 strain.

### [Example 6]

Genetic analysis and gene marker development were carried out in order to specify the chromosome location region of the RTM-1 gene.

First, the Viroflay line and the TNKH-1 line developed in Example 1 were crossed, and the thus obtained F1 seeds were self-pollinated to produce an F2 segregating population.

From this F2 segregating population, 330 individuals were subjected to inoculation tests using the race indicated by UA1014 strain to investigate resistance to this race. The results revealed 234 resistant individuals and 96 susceptible individuals.

Genetic analysis of these individuals was then conducted, and a search was conducted for the region within the genome responsible for resistant homozygosis or heterozygosis in the resistant individuals, and the region within the genome responsible for susceptible homozygosis in the susceptible individuals.

Individuals that had undergone investigation of their traits were used as a linkage analysis population, and genomic DNA was extracted from each individual. Genotyping was conducted using the DNA from the linkage analysis population and SNP markers designed for the SpinachBase reference genome. The genotyping was conducted using KASP genotyping assays.

The nucleotide sequences of primers used in the KASP genotyping assays used for identifying each SNP are shown in Tables 8 and 9. In Tables 8 and 9, the "A allele" indicates the TNKH-1 (Spinacia tetrandra) allele, and the "B allele" indicates the Viroflay allele. The genotype of each SNP was identified using a three-primer set composed of an allele specific primer_1, an allele specific primer _2, and a common primer. In the sequences of the allele specific primer_1 and the allele specific primer_2, one base at the 3' end is designed to hybridize with the identification target SNP.

When the genomic DNA of a plant individual having the TNKH-1 allele is used as a template, an amplified product is obtained by PCR using the allele specific primer_1 and the common primer as the forward primer and reverse primer, respectively. When the genomic DNA of a plant individual having the Viroflay allele is used as a template, an amplified product is obtained by PCR using the allele specific primer_2 and the common primer as the forward primer and reverse primer, respectively. The presence or absence of these amplified products is used to identify the SNP genotypes. By conducting a KASP assay in this manner, using a single primer set containing these three types of oligonucleotide DNA, the genotype of each SNP can be identified.

**[Table 8]**

| SNP | | | | Allele | SEQ ID NO: |
|---|---|---|---|---|---|
| chr4_ 7421480 | Reverse strand | allele specific primer_1 | GAAGGTGACCAAGTTCATGCTATCCCCGG CTTTCTTATCCTG | A | 1 |
| | | allele specific primer_2 | GAAGGTCGGAGTCAACGGATTATCCCCGG CTTTCTTATCCTC | B | 2 |
| | | common primer | CATGTGAAGGAAGGGACACCA | | 3 |
| chr4_ 7962907 | Reverse strand | allele specific primer_1 | GAAGGTGACCAAGTTCATGCTATGCTCGT ATACGCAATGGC | A | 4 |
| | | allele specific primer_2 | GAAGGTCGGAGTCAACGGATTCATGCTCG TATACGCAATGGT | B | 5 |
| | | common primer | CCAATTGACCAGAATTGTCACC | | 6 |
| chr4_ 8152986 | Forward strand | allele specific primer_1 | GAAGGTGACCAAGTTCATGCTGGGAGCA ACATTTATGCAAGAC | A | 7 |
| | | allele specific primer_2 | GAAGGTCGGAGTCAACGGATTGGGAGCA ACATTTATGCAAGAT | B | 8 |
| | | common primer | ATGTATGGTCTGACAACGGCA | | 9 |
| chr4_ 8190990 | Reverse strand | allele specific primer_1 | GAAGGTGACCAAGTTCATGCTATCGGGAG GAAAGCTCTAACC | A | 10 |
| | | allele specific primer_2 | GAAGGTCGGAGTCAACGGATTATCGGGAG GAAAGCTCTAACT | B | 11 |
| | | common primer | TGTGTACCCATATTGGCCAGA | | 12 |
| chr4_ 8488603 | Reverse strand | allele specific primer_1 | GAAGGTCGGAGTCAACGGATTTAATGGGA TCTGCTGCCTCG | A | 13 |
| | | allele specific primer_2 | GAAGGTGACCAAGTTCATGCTCTAATGGG ATCTGCTGCCTCA | B | 14 |
| | | common primer | CAAAGGCTTGACGGTCCAGTA | | 15 |

**[Table 9]**

| SNP | | | | Allele | SEQ ID NO: |
|---|---|---|---|---|---|
| chr4_ 8494600 | Reverse strand | allele specific primer_1 | GAAGGTGACCAAGTTCATGCTTCTCGCTTG TCATTGTCAGCA | A | 16 |
| | | allele specific primer_2 | GAAGGTCGGAGTCAACGGATTTTCGCTTG TCATTGTCAGCG | B | 17 |
| | | common primer | CTGTGGAGTCGGAAGAACACA | | 18 |
| chr4_ 8510715 | Forward strand | allele specific primer_1 | GAAGGTGACCAAGTTCATGCTCTGGTCCAT ACTCATACTCAGGG | A | 19 |
| | | allele specific primer_2 | GAAGGTCGGAGTCAACGGATTCTGGTCCA TACTCATACTCAGGC | B | 20 |
| | | common primer | AGCTAATTCTGAGCCAAGGCC | | 21 |
| chr4_ 8617232 | Forward strand | allele specific primer_1 | GAAGGTCGGAGTCAACGGATTTGAGACAG CGGGATAAAGAGTAAG | A | 22 |
| | | allele specific primer_2 | GAAGGTGACCAAGTTCATGCTTGAGACAG CGGGATAAAGAGTAAA | B | 23 |
| | | common primer | TCAGTTCGACAAGCTGCCTAG | | 24 |
| chr4_ 10710358 | Forward strand | allele specific primer_1 | GAAGGTCGGAGTCAACGGATTCGAGTTGA TCACCTCGTCG | A | 25 |
| | | allele specific primer_2 | GAAGGTGACCAAGTTCATGCTCGAGTTGA TCACCTCGTCC | B | 26 |
| | | common primer | TGTACCCAGTTGTCGAGCC | | 27 |

For example, the TNKH-1 line has homozygous alleles in which the genotype of the SNP specified by chr4_8510715 is G (guanine), whereas Viroflay has homozygous alleles in which the genotype of the SNP specified by chr4_8510715 is C (cytosine). Further, in an F1 of the TNKH-1 line and Viroflay , the genotype of the SNP specified by chr4_8510715 is G/C, with the TNKH-1 allele in which the SNP is G and the Viroflay allele in which the SNP is C existing in a heterozygous manner.

The KASP markers used in the linkage analysis are used for the purpose of specifying the RTM-1 gene locus, but these KASP markers need not necessarily be used as the DNA markers when selecting spinach plants having downy mildew resistance. For example, DNA polymorphisms other than the SNPs listed in Table 8 and Table 9, but located within the spinach genome range specified by chr4_7962907 to chr4_8617232, or DNA polymorphisms that exhibit a strong correlation with the downy mildew resistance trait derived from the gene locus located at chr4_7962907 to chr4_8617232 may also be used as DNA markers when selecting spinach plants having downy mildew resistance.

By using genomic DNA extracted from individual plants and the KASP markers listed in Tables 8 and 9, the genotype of each SNP in the linkage analysis population was able to be examined with ease. Following acquisition of this genotyping data using the KASP markers, linkage analysis was conducted using the ultra-high density genetic linkage map creation software AntMAP Ver 1.1 (http://lbm.ab.a.u-tokyo.ac.jp/~iwata/antmap/) (Non-Patent Document 5). When using this software all criteria were set to their defaults except for altering the Grouping criterion to Distance (cM) / Haldane / Threshold 5.

Table 10 displays the genotypes together with the phenotype for seven individuals (sample numbers 112/143/322/294/53/232/6) in which, based on the results of the genotyping using the KASP markers illustrated in Tables 8 and 9, a substitution has occurred in a peripheral region to the RTM-1 gene. In the table, "A" indicates the TNKH-1 allele, and "B" indicates the Viroflay allele. For example, "A/A" indicates an individual having TNKH-1 alleles in a homozygous manner, "B/B" indicates an individual having Viroflay alleles in a homozygous manner, and "A/B" indicates an individual having the TNKH-1 allele and the Viroflay allele in a heterozygous manner.

**[Table 10]**

| | Genotype | | | | | | | Genetic distance from phenotype [cM] |
|---|---|---|---|---|---|---|---|---|
| Sample number | 112 | 143 | 322 | 294 | 53 | 232 | 6 | |
| Phenotype | R | R | R | R | R | S | S | |
| chr4_7421480 | B/B | B/B | B/B | B/B | A/B | A/B | B/B | 5.5 |
| chr4_7962907 | A/B | A/A | A/B | B/B | A/B | B/B | B/B | 2.1 |
| chr4_8152986 | A/B | A/A | A/B | B/B | A/B | B/B | B/B | 1.8 |
| chr4_8190990 | A/B | A/A | A/B | B/B | A/B | B/B | B/B | 1.8 |
| chr4_8488603 | A/B | A/B | A/B | A/B | A/B | B/B | B/B | 0.0 |
| chr4_8494600 | A/B | A/B | A/B | A/B | A/B | B/B | B/B | 0.0 |
| chr4_8510715 | A/B | A/B | A/B | A/B | A/B | B/B | B/B | 0.0 |
| chr4_8617232 | A/B | A/B | A/B | B/B | A/B | B/B | B/B | 0.2 |
| chr4_10710358 | A/B | A/B | A/B | B/B | B/B | B/B | A/B | 4.1 |

As illustrated in Table 10, based on the fact that for the SNPs of chr4_8488603, chr4_8494600 and chr4_8510715, the two samples of sample 232 and sample 6 in which the genotype was B/B had a phenotype of S (susceptible), whereas the sample 112, the sample 143, the sample 322, the sample 294 and the sample 53 in which the genotype was A/B had a phenotype of R (resistant), it was evident that these SNPs exhibited a strong correlation with the phenotype. Further, based on the results of the linkage analysis, it was clear that in the SpinachBase reference genome, the RTM-1 gene that conveys downy mildew resistance was located within the range from chr4_7962907 to chr4_8617232. Further, the SNPs of chr4_8488603, chr4_8494600 and chr4_8510715 were linked with the RTM-1 gene at a genetic distance of 0 cM (FIG. 1).

For the Spinacia tetrandra line CGN25466:MGK 01, the TNKH-1 line and the TNKH-2 line, a SNP search was conducted from the sequence analysis data of the entire nucleotide sequence within the range from chr4_7962907 to chr4_8617232 where the RTM-1 gene is located. The results confirmed a plurality of SNPs other than the SNPs listed in Table 8 and Table 9. Among these other SNPs, for example, the four SNPs of chr4_8500200, chr4_8502319, chr4_8502334 and chr4_8502394 differed from the reference genome. Specifically, in the Spinacia tetrandra line CGN25466:MGK 01, the TNKH-1 line and the TNKH-2 line, the SNP specified by chr4_8500200 was thymine, the SNP specified by chr4_8502319 was cytosine, the SNP specified by chr4_8502334 was guanine, and the SNP specified by chr4_8502394 was guanine (Table 1). In a similar manner to the SNPs shown in Table 8 and Table 9, these four SNPs can be used as markers for identifying downy mildew resistant plant individuals having a TNKH-1 allele.

### [Accession Numbers]

FERM BP-22404
FERM BP-22405

### [Sequence Listing]

PC34414_WA_sequences.txt

## Claims

1. A downy mildew resistant spinach plant (excluding Spinacia tetrandra) having a downy mildew resistance RTM-1 gene, which is located on chromosome 4 of the Spinacia tetrandra line CGN25466:MGK 01.

2. The downy mildew resistant spinach plant according to Claim 1, wherein the RTM-1 gene is a gene that exists in a range from chr4_7962907 to chr4_8617232 on chromosome 4 of the Spinacia tetrandra line CGN25466:MGK 01.

3. The downy mildew resistant spinach plant according to Claim 1 or 2, wherein at least one allele of chromosome 4 contains a fragment that includes a range from chr4_8488603 to
chr4_8510715 on chromosome 4 of the Spinacia tetrandra line CGN25466:MGK 01.

4. The downy mildew resistant spinach plant according to Claim 1, wherein the RTM-1 gene is either homozygous or heterozygous.

5. The downy mildew resistant spinach plant according to Claim 1, wherein the plant is resistant to at least downy mildew races Pfs1, Pfs2, Pfs3, Pfs4, Pfs5, Pfs6, Pfs7, Pfs8, Pfs9, Pfs10, Pfs11, Pfs12, Pfs13, Pfs14, Pfs15, Pfs16, Pfs17, Pfs18 and Pfs19, and a race indicated by UA1014 strain.

6. The downy mildew resistant spinach plant according to Claim 1, wherein in at least one allele of chromosome 4:
a SNP specified by chr4_8488603 is cytosine,
a SNP specified by chr4_8494600 is thymine, or
a SNP specified by chr4_8510715 is guanine.

7. The downy mildew resistant spinach plant according to Claim 1, wherein in at least one allele of chromosome 4:
a SNP specified by chr4_7962907 is guanine,
a SNP specified by chr4_8152986 is cytosine,
a SNP specified by chr4_8190990 is guanine,
a SNP specified by chr4_8488603 is cytosine,
a SNP specified by chr4_8494600 is thymine,
a SNP specified by chr4_8510715 is guanine, or
a SNP specified by chr4_8617232 is guanine.

8. The downy mildew resistant spinach plant according to Claim 1, wherein the downy mildew resistant spinach plant is derived from an interspecific hybrid plant of Spinacia tetrandra and a cultivated variety of spinach.

9. The downy mildew resistant spinach plant according to Claim 1, having downy mildew resistance derived from a plant specified in accession number FERM BP-22404 (TNKH-1 line).

10. The downy mildew resistant spinach plant according to Claim 1, having downy mildew resistance derived from a plant specified in accession number FERM BP-22405 (TNKH-2 line).

11. The downy mildew resistant spinach plant according to Claim 1, having at least one type of downy mildew resistance gene besides the RTM-1 gene.

12. A downy mildew resistant spinach plant that is a downy mildew resistant spinach plant specified in accession number FERM BP-22404 (TNKH-1 line), a hybrid plant obtained using the downy mildew resistant spinach plant as a parent, or a progeny of either of these plants.

13. A downy mildew resistant spinach plant that is a downy mildew resistant spinach plant specified in accession number FERM BP-22405 (TNKH-2 line), a hybrid plant obtained using the downy mildew resistant spinach plant as a parent, or a progeny of either of these plants.

14. A method for predicting downy mildew resistance of a spinach plant, the method comprising investigating genotypes of SNPs from chr4_8488603 to chr4_8510715 of chromosome 4 of a test spinach plant, and predicting that the test spinach plant will have a high probability of exhibiting downy mildew resistance in those cases where, in at least one allele:
a SNP specified by chr4_8488603 is cytosine,
a SNP specified by chr4_8494600 is thymine, or
a SNP specified by chr4_8510715 is guanine.

15. A method for screening downy mildew resistant spinach plants, the method comprising investigating genotypes of SNPs from chr4_8488603 to chr4_8510715 of chromosome 4 of a test spinach plant, and selecting the test spinach plant as a downy mildew resistant spinach plant in those cases where, in at least one allele:
a SNP specified by chr4_8488603 is cytosine,
a SNP specified by chr4_8494600 is thymine, or
a SNP specified by chr4_8510715 is guanine.

16. A method for producing a downy mildew resistant spinach plant, the method having:
a first crossing step of crossing a spinach plant having an RTM-1 gene and an arbitrary spinach plant,
a second crossing step of obtaining a segregating population by subjecting an F1 individual obtained in the first crossing step to self-pollination, backcrossing, or interspecific crossing or intraspecific crossing with a spinach plant different from the parent used in the first crossing step, and
a selection step of selecting a spinach plant having an RTM-1 gene from the segregating population.

17. The method for producing a downy mildew resistant spinach plant according to Claim 16, wherein the spinach plant having an RTM-1 gene is Spinacia tetrandra or a downy mildew resistant spinach plant according to any one of Claims 1 to 13.

18. The method for producing a downy mildew resistant spinach plant according to Claim 16, wherein the arbitrary spinach plant, or the parent used in the interspecific crossing or intraspecific crossing in the second crossing step is a spinach plant having at least one type of downy mildew resistance gene besides the RTM-1 gene.

19. A portion of a plant body of the downy mildew resistant spinach plant according to any one of Claims 1 to 13.

20. A leaf of the downy mildew resistant spinach plant according to any one of Claims 1 to 13.

21. A seed of the downy mildew resistant spinach plant according to any one of Claims 1 to 13.

22. A DNA marker for selecting a spinach plant having an RTM-1 gene, wherein the DNA marker is composed of at least one SNP selected from the group consisting of a SNP specified by chr4_8488603 of the spinach plant, a SNP specified by chr4_8494600 of the spinach plant, a SNP specified by chr4_8510715 of the spinach plant, and SNPs strongly linked to these SNPs.

23. A kit which is used for selecting a spinach plant having an RTM-1 gene, the kit comprising at least one type of primer set selected from the group consisting of:
a primer set for identifying a genotype of a SNP specified by chr4_8488603 of the spinach plant,
a primer set for identifying a genotype of a SNP specified by chr4_8494600 of the spinach plant, and
a primer set for identifying a genotype of a SNP specified by chr4_8510715 of the spinach plant.

24. The kit according to Claim 23, wherein
the primer set for identifying a genotype of the SNP specified by chr4_8488603 includes a primer composed of a nucleotide sequence represented by SEQ ID NO: 13, a primer composed of a nucleotide sequence represented by SEQ ID NO: 14, and a primer composed of a nucleotide sequence represented by SEQ ID NO: 15,
the primer set for identifying a genotype of the SNP specified by chr4_8494600 includes a primer composed of a nucleotide sequence represented by SEQ ID NO: 16, a primer composed of a nucleotide sequence represented by SEQ ID NO: 17, and a primer composed of a nucleotide sequence represented by SEQ ID NO: 18, and
the primer set for identifying a genotype of the SNP specified by chr4_8510715 includes a primer composed of a nucleotide sequence represented by SEQ ID NO: 19, a primer composed of a nucleotide sequence represented by SEQ ID NO: 20, and a primer composed of a nucleotide sequence represented by SEQ ID NO: 21.
